**Europäisches Patentamt**

**European Patent Office**

**Office européen des brevets**

(11) Publication number: **0 130 015**
**B1**

# EUROPEAN PATENT SPECIFICATION

(45) Date of publication of patent specification: **23.08.89**

(51) Int. Cl.⁴: **A 61 B 5/10**

(21) Application number: **84304036.1**

(22) Date of filing: **15.06.84**

(54) Compact fingerprinting system.

(30) Priority: **23.06.83 US 507283**

(43) Date of publication of application:
**02.01.85 Bulletin 85/01**

(45) Publication of the grant of the patent:
**23.08.89 Bulletin 89/34**

(84) Designated Contracting States:
**AT BE CH DE FR GB IT LI LU NL SE**

(56) References cited:
**GB-A- 428 386**
**US-A-3 089 459**
**US-A-4 379 178**

(73) Proprietor: **DACTEK INTERNATIONAL, INC.**
**8101 Orion Avenue, Suite 19**
**Van Nuys California 91406 (US)**

(72) Inventor: **Meadows, Louis B.**
**25503 Via Jardin**
**Valencia California 91355 (US)**
Inventor: **Diamond, Arthur S.**
**9850 Old Creek Road**
**Ventura California 93001 (US)**

(74) Representative: **Thomson, Paul Anthony et al**
**Potts, Kerr & Co. 15, Hamilton Square**
**Birkenhead Merseyside L41 6BR (GB)**

Courier Press, Leamington Spa, England.

## Description

### Technical field

The present invention relates to an inkless fingerprint identification system and, more particularly, to a compact system for directly imaging fingerprints on a fingerprint card from a fingerprint moistened with a non-greasy marking composition applied from a compact applicator.

### Background art

Since fingerprint patterns of ridge endings and ridge bifurcations do not vary with time for an individual and the pattern on each finger for any individual is unique and differentiates that individual from the rest of society, fingerprint comparison is an absolute means of identification. This fact has been accepted by the scientific community and by the courts. Fingerprint identification is legally recognized forensic evidence of an individual's presence at a scene or association with property or instruments used in a crime. Fingerprint identification is also used commercially such as on identification cards for security systems, check identification means, and in many non-criminal government agencies. Fingerprint identification is coming into use as a means of identifying and locating missing children and footprint identification is in almost universal usage in developed countries for infant identification.

Any reliable fingerprint identification system requires imaging a distinct print pattern on a substrate. Even in commercial identification systems such as with checks, it is important that the system be inoffensive to the subject. The early inking systems were based on greasy black inks. These systems were difficult to utilize since it required much skill, training and care to provide a distinct print without ink running between ridges and obliterating substantial areas of the print image. Furthermore, the inking system was messy to use for both the operator and the subject, requiring towelettes or a trip to a wash basin in order to remove the ink.

### Description of the prior art

U.S. Patent No. 3,089,459 relates to a fingerprint marking composition applicator comprising a vapor-proof enclosure and a porous pad contained within the enclosure and impregnated with a controlled amount of marking composition. The enclosure is an edge-sealed, flexible packet and the print of a foot or finger pattern is identifiable and can be used for identification purposes.

There have been several different inkless fingerprint systems proposed, such as a magnetizable powder system disclosed in U.S. Patent No. 3,831,552, or various imaging systems based on reaction between metal salts and polyhydroxy aromatic compounds. One very convenient implementation of this imaging reaction is application of a thin, transparent coating from a pad impregnated with one of the metal salts, such as an iron chloride.

A substantially invisible fingerprint transferred to a check or fingerprint card is developed by spraying developer onto the invisible fingerprint pattern. The metallic salt on the card and the spray applied organic developing compound quickly react to form a colored compound which renders the fingerprint visible to form a permanent record.

An improved system disclosed in our U.S. Patent No. 4,379,178, avoids the use of fluorocarbon propellant based sprays by impregnating the developer directly into the fingerprint card or other fibrous substrate. However, though this system provides a substantial improvement in ease of developing the card, it still requires the utilization of an impregnated porous, plaster pad to apply the marking compound.

The impregnating solution includes hydroscopic compounds such as glycerin. In periods of high humidity the pad absorbs excessive moisture resulting in blurred and smeared prints, unless the top of the pad is first blotted with a towel or tissue. The pad can absorb so much moisture that the solution will overflow and run over the edges of the container. In periods of low humidity, the water will evaporate from the impregnating solution and the pad becomes too dry to use. Prints are again of poor quality due to incomplete and light print formation unless water is added to the pad.

There are many fingerprinting programs that would be greatly expanded in scope if a simpler, more reliable system could be developed and, especially if a compact system was available that was simple to operate and could be sent through the mails.

### Statement of the invention

According to the present invention there is provided a fingerprint identification system comprising a fingerprint marking composition applicator in the form of a thin packet comprising a vapor-proof enclosure and a porous pad contained within the enclosure impregnated with a controlled amount of marking composition; and a substrate in the form of a fingerprint card having a portion impregnated with a color developer for the marking composition; characterised in that the packet is formed on a portion of the card.

A compact fingerprinting system that is insensitive to the environment is provided in accordance with the invention. The moisture content of the marking composition applicator is precisely controlled and is at optimum concentration at the time of use. The system is capable of use by the non-expert and can be administered by any user. The components of the system are light in weight, small, thin, and can be mailed to the user and mailed back to the fingerprint registering agency after operating the system to prepare a set of prints.

Since the system is ready to use without the necessity to adjust moisture content, it is a preferred system for use in delivery rooms for securing foot-prints of infants. The compact system of the invention can be made of sterile components which are more compatible with septic conditions in a delivery room, than is an aqueous impregnated plaster pad or a greasy ink roller which are handled by many users and exposed to micro-organisms in the environment.

The identification system of the invention provides a dark, distinct print image as a permanent record directly on a card pre-coated with developer in a simple, efficient and reliable manner, and uses techniques and materials familiar to operators of fingerprinting systems. The fingerprint image develops rapidly and distinctly and forms a permanent record for use in any of the security or forensic procedures previously practiced in this field.

A print is formed in the system of the invention by opening a sealed packet containing a porous pad impregnated with a solution of a color-forming, water-soluble, metal salt marking composition, and impressing the distal portion of a finger or other body part onto the pad to form a latent, transparent pattern. The pattern is transferred to a card and the marking compound in the pattern reacts with a developer to form a dark, distinct, permanent image. The developer can be applied as a spray, as disclosed in U.S. Patent No. 3,960, 632, or preferably by impregnating the developer onto a fingerprint card, as disclosed in U.S. Patent No. 4,379,178, the disclosures of which are hereby expressly incorporated herein by reference.

The pad always contains an optimum concentration of marking composition, since it is isolated from effects of dry, wet or hot environments by the sealed envelope in which it is stored. There is no need to premoisten the finger tip with a solution of detergent, as suggested in U.S. Patent No. 4,379,178, since dark, distinct prints are provided by developing the optimum concentration pattern of marking composition provided in the system of the invention. The sealed package also provides a more septic applicator for use in sterile environments, such as a delivery room. The package is opened exposing the pad, and the finger is lightly rubbed or pressed on the pad to form a thin film, latent image pattern. The pad can be reused several times for different fingers or for several different subjects over an extended period of several hours or days depending on ambient conditions, before it is disposed. The sealed pad is light and compact, and when combined with a card precoated with developer forms a light weight, thin package that is suitable for mailing to and from individual users.

In the accompanying drawings, Figures 4 and 5 are in accordance with the present invention and Figures 1 to 3 and 6 and 7 are merely given by way of information, and are not in accordance with the present invention.

Brief description of the drawings

Figure 1 is a front view in elevation of a sealed applicator;

Figure 2 is a view in section taken along line 2—2 of Figure 1;

Figure 3 is a side view in elevation of another embodiment of a sealed applicator;

Figure 4 is a front view in elevation of a card with a sealed applicator section in accordance with the invention;

Figure 5 is a view in section taken along line 5—5 of Figure 4;

Figure 6 is a front view in elevation of a packaged identification system; and

Figures 7A, 7B and 7C are schematic views of the steps practiced in forming a fingerprint with the system of Figure 6.

Detailed description of the invention

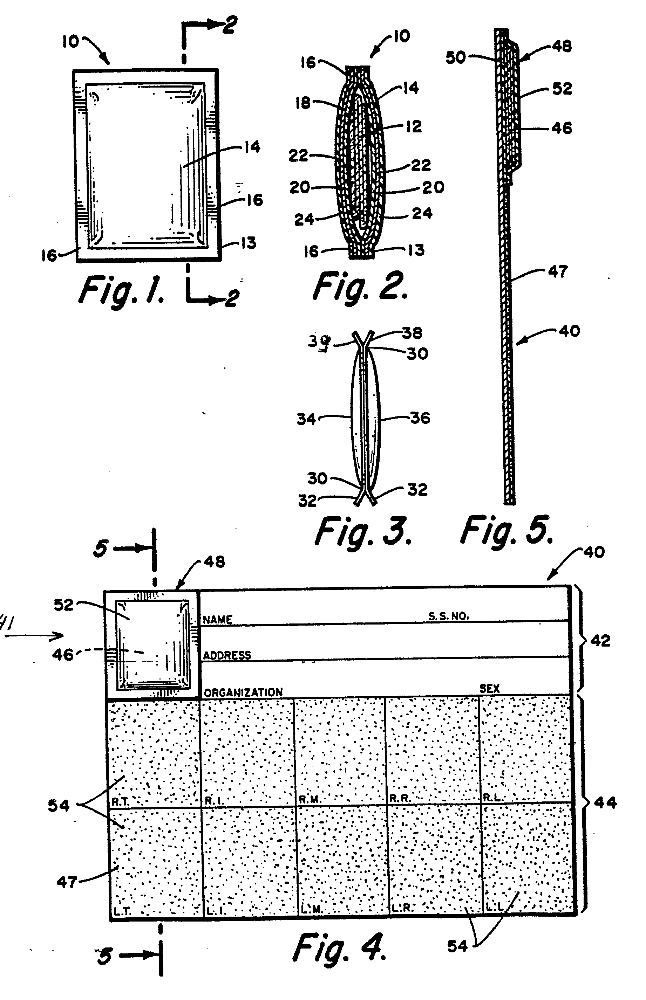

Referring now to Figures 1 and 2, the sealed applicator 10 comprises a porous pad 12 impregnated with marking composition sealed with an enclosure 13 that has a very low moisture vapor transmission rate under normal ambient storage conditions, such as from −20°F to 115°F (−29°C to 46°C). The water vapor permeability as measured by ASTM D697 method is below 0.5 and preferably below 0.2 grams/24 hr/mm thickness/cc Hg at 25°C. The enclosure is preferably formed of a soft, flexible film, such as a low water vapor transmission resin or a thin foil metal, such as aluminum and preferably a combination thereof. A flat, edge-sealed packet as best illustrated in Figure 2 is a particularly preferred form of the enclosure.

The packet is formed of a top sheet 14 attached along its four edges by means of a seal 16 to a bottom sheet 18. The seal 16 can be formed by a wet adhesive, but is preferably formed by applying a layer 20 of a thermoplastic, such as polyethylene or polypropylene to the inner surfaces of a central vapor barrier film 22, such as a sheet of aluminum foil, and applying heat to fuse the layers 20 together. The outer surfaces of the aluminum foil can contain a layer 24 of a tougher resin, such as a Nylon (polyamide) or Mylar (linear polyester) to provide resistance to wrinkling or cracking, and to provide a surface for imprinting product name or instructions. The coil can be replaced with a synthetic resin film having good vapor barrier characteristics, such as polyvinylidene fluoride.

The porous pad 12 is a hydrophilic material which is impregnated with the marking composition. The pad should not have a surface capable of imprinting its pattern on a fingertip, such as a sponge with pores larger than ridge separations on a finger, or a stiff, textured paper. Best results are achieved with stretchable, creped paper towelettes. The important characteristic is the amount of solution impregnated into the paper. For 5 inch by 8 inch (12.7 cm by 20.3 cm) (40 square inches) (258 sq. cm.) folded, paper towelettes, it has been found that at an imprengation of 1.66 cc of marking solution or less, the print is incomplete and light. At an impregnation of 2.2 cc of marking solution or more, the print smudges or smears. About 1.8 cc of liquid per 40

square inches appears to be optimum.

The packet shown in Figure 3 is easier to open, since the seam lines 30 are displaced inward from the outer edges 32 of the upper and lower foil laminate sheets 34, 36 by about 1/16 to 1/4 inch. This leaves a set of lips 38, 40 which can be gripped to part the side seams.

The marking composition impregnated into the pad comprises a solution of a water-soluble, metal salt, marking compound and a water-soluble, solvent-lubricant. The composition may also contain a small amount of a wetting agent or detergent. The solution generally contains, on a relative basis, 20 to 100 parts by weight of solvent; 2 to 30 parts by weight of metal salt; and optionally, 0 to 20 parts of water. A small amount of detergent, such as 1 to 10 grams of Aerosol OT (75% AQ), may be added. The ingredients are mixed to form a clear solution which is then soaked into the pad. The solution enters the pores of the paper pad.

The soluble metal salt reactive with the hydroxyphenolic develolper can be a metal from groups I to VIII of the periodic table, and the anion may be inorganic, such as halide, sulfate or ferrocyanide. A preferred marking ingredient, due to cost, availability, nontoxicity and safety, is ferric chloride. Ferric chloride may be used in a mixture with 5 to 30% of its weight of ferrous chloride. The solvent for the salt is preferably a liquid that does not evaporate under ambient conditions, and also preferably is a lubricant to lubricate the movement of the finger as it moves over the paper pad. Preferred solvent-lubricants are materials, such as glycerine, an alkylene glycol, such as ethylene glycol or propylene glycol, or various low molecular weight polyether liquids based on ethylene and/or propylene oxide. A suitable example of a pad soaking solution follows:

### Example 1

| Material | Amount |
|---|---|
| Glycerine | 23,866 grams |
| FeCl$_3$ · 6H$_2$O | 5,818 grams |
| FeCL$_2$ · 4H$_2$O | 763 grams |
| Aerosol OT | 8 ml |

The hydroxyaromatic developer compound that forms the marking reaction with the metal salt is impregnated into a substrate, suitably a fibrous substrate, such as a paper check or ID card 40 shown in Figure 4, 5, or 6, by impregnation from solution. The card 40 will have a data receiving area 42 on the top portion 44 in the lower half thereof. The marking solution and a fingerprint receiving area need only be impregnated into the fingerprint portion 44 of the card to form an impregnation layer 47.

The metal salt is preferably a salt of a transition metal, such as iron, titanium, vanadium, chromium, magnesium, cobalt, nickel, copper, molybdenum, tungsten and the like with an anion, such as ferride, citrate, sulfate, nitrate, stearate, acetate, formate, phosphate and the like.

The preferred developing ingredients are quinolinol derivatives, preferably 8-hydroxyquinoline and various substitute derivatives thereof alone or in combination with a polyhydroxy phenol compound, such as trihydroxy benzoic acid, pyrrogallol, catechol, gallic acid, propyl gallate, and the like. The developing reaction should be such as to give a clear and distinct image, preferably a very dark, black-colored image. The impregnating composition is formed as a solution in a common solvent. Solvents, such as acetone are utilizable; however, for inhalation reasons and due to the tendency of acetone to dissolve preprinted areas of the fingerprint card, it is preferred to utilize an alcohol solvent, suitably a lower alkanol, such as methanol, ethanol or mixtures thereof. The developing composition contains, based on 100 grams of solvent, 10 to 40 parts by weight of marking compound, and 1 to 10 parts of the higher molecular weight dibasic/acid additive of the invention. The composition may also contain from 0.1 to 3 parts of a finely divided silica as a thickener. The preferred composition contains a mixture of a trihydroxybenzene, such as propyl gallate and 8-hydroxyquinoline in a ratio of at least five to one of the gallate to the hydroxy-quinoline, preferably at least ten to one. The preferred dibasic acid is azelaic acid. A card impregnating solution is made by heating the solvent gently with stirring to dissolve ingredients while maintaining a maximum temperature of 45° until the azelaic acid is dissolved, then removing the heat and adding the finely divided silica, such as Cab-O-Sil, if desired.

A suitable example of practice follows.

### Example 2

| Material | Amount |
|---|---|
| Denatured alcohol | 1540 ml |
| Propyl gallate | 240 grams |
| 8-Hydroxy-quinoline | 15.75 grams |
| Azelaic acid | 60 grams |
| Cab-O-Sil M5 | 7.5 grams |

The cards are coated with this solution or preferably imprinted by means of the water fountain of an offset press on a basis of 0.01 to 10 pounds (0.0045 to 4.5 kg) of impregnating solution for 3,000 square feet (270 sq. m) of cards. It has been determined that for normal cards and good imaging, the coating basis can generally be 0.5 to 1.0 pounds (0.23 to 0.45 kg) per 3,000 square feet (270 sq. m) of cards.

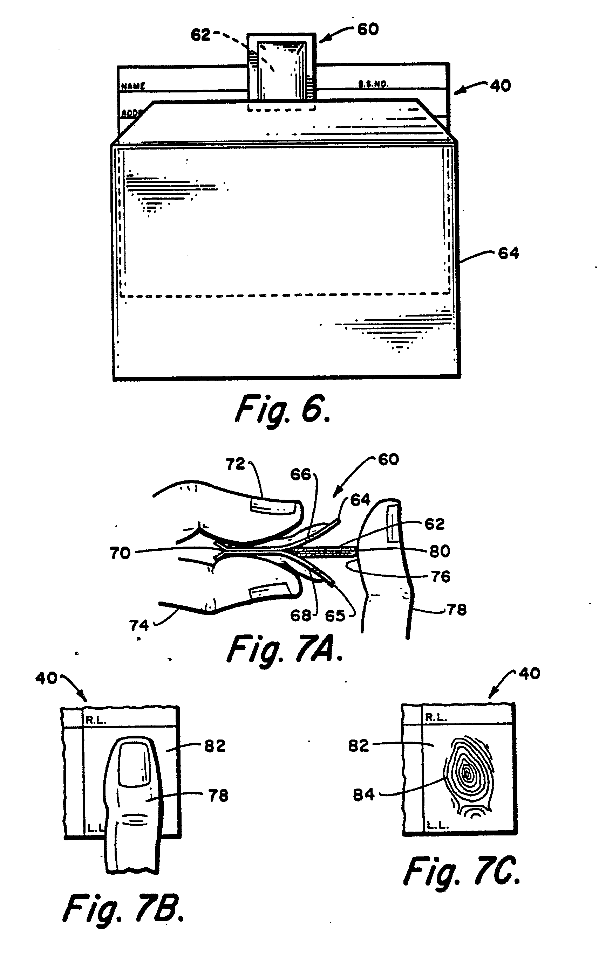

Figures 4 and 5 illustrate a fingerprint card 40 and an applicator 41 containing an impregnated pad 46. The pad 46 is placed in a sealed, vaporproof envelope 48 formed by means of a bottom film 50, edge-sealed to a peelable upper film 52. The film 52 is cut or pulled to expose the pad 46. A finger is pressed onto the pad 46 to form a pattern, and the pattern is then pressed onto a premarked square 54 of the coated portion 44.

Referring now to Figures 6 and 7, a compact mailable identification system includes a sealed packet 60 containing an impregnated pad 62, and a developer impregnated fingerprint card 40 enclosed in an envelope 64. The card may contain a preprinted return address on its rear surface. The system is mailed to a user who removes the card 40 and fills in the requested personal data information in area 42. The user then opens packet 60 by grabbing lips 64, 65 and splitting the side seams 66, 68 and pressing the rear 70 of the packet with his thumb 72 and forefinger 74 to slide the pad 62 partially out of the packet 60. As shown in Figure 7A, he then slides the distal portion 76 of one of his fingers 78 along the folded edge 80 of the pad 62 to form a pattern.

He then presses the finger 78 onto the appropriate square 82 on the lower portion 44 of the card 40 as shown in Figure 7B. A dark, distinct fingerprint 84 develops instantly as shown in Figure 7C.

The following examples were prepared and tested. The packets contained various amounts of solution of Example 1 and the indicated amount of water dilution impregnated onto 40 square inches (5 inch×8 inch) (12.7 cm×20.3 cm) folded paper. The packet size was about 2 inches square (5 cm square). The following procedure was followed:

1. Tear packet open across the top.

2. Squeeze pad out of packet so that only about 1/4 inch (0.63 cm) is exposed. Do not remove pad completely—just squeeze it out of the packet as you would squeeze toothpaste from a tube.

3. Apply solution evenly to thumb (or index finger) of right hand.

4. Roll a print onto the Dactek treated card.

5. Repeat steps 3 and 4 for the thumb or index finger of your left hand.

Example 3

1.6 ml of the solution of Example 1 containing 10% water was impregnated into a folded 5 inch×8 inch (12.7 cm×20.3 cm) creped paper and sealed into a packet.

Example 4

Example 3 was repeated utilizing a folded 5 inch×8 inch (12.7 cm×20.3 cm) bonded, non-woven paper as the pad.

The creped paper pad of Example 3 was saturated uniformly throughout and provided a very clear, uniform print with fine detail throughout. It did appear to be somewhat dry. There was no noticeable difference in color when compared to control (a plaster pad impregnated with the same solution as in Examples 3 and 4) though the print of Example 3 was slightly lighter in density than the control print, or the print of Example 4. The pad in the packet of Example 4 was not saturated uniformly. It was too wet on the edges and completely dry in the center. It gave a blotchy print with much of the detail completely obscured.

A further series of experiments were conducted utilizing a folded 5 inch×8 inch (12.7 cm×20.3 cm) creped paper (22 pound (9.9 kg) basis) and varying the level of fill, and the amount of water to determine the effect on color, density and quality of resulting fingerprints.

TABLE 1

| Fill (ccs) | Solution of Example 1 |
|---|---|
| 2.2 | 100 |
| 2.0 | 100 |
| 1.8 | 100 |
| 2.2 | 95 |
| 2.0 | 95 |
| 1.8 | 95 |
| 1.8 | 95 |
| Control | 100 |

The following results were obtained.

TABLE 2

| Pad feel | Fingerprint quality |
|---|---|
| Sticky, too wet | Dark, slightly blotchy |
| Sticky, OK | Good |
| Too dry | Light |
| Too wet | Good, a bit uneven |
| OK | Better, a bit uneven |
| OK | Very good |
| OK | Very good |
| OK | Dark, a bit uneven |

As a result of these tests, it is concluded that the best fill is in the range of 1.8 to 2.0 ccs per 40 square inches (100 sq. cm) of the 22-pound (9.9 kg) crepe paper, using 95% solution and 5% water. On a weight basis, 1.8 to 2.0 ccs per 40 square inches (100 sq. cm) equates to 383 to 425 ccs of solution per pound of (0.45 kg) of paper. At a specific gravity of 1.0, this is from 84.4 to 93.7 pounds (38.78 to 42.5 kg) of solution per 100 pounds (45 kg) of dry paper. Actually, the specific gravity is about 1.5, making the range 127 to 141 pounds (57.6 to 64 kg) of solution per 100 pounds (45 kg) of dry paper. The degree of saturation is critical. Below 127% (of dry paper weight) saturation, the towelette is too dry and not enough reagent transfers to the finger. The print comes out too light. Above the 141% saturation, the towelette is too wet; an excess of solution is picked up and prints appear blotchy.

Performance (fingerprint quality) is also influenced by the amount of water present in the impregnating solution. At 100% concentration, the towelettes feel "sticky". The solution is too viscous and transfers unevenly to the finger. At 95% concentration, the solution does not feel sticky, and a smooth, even transfer results.

The card and packet weigh less than two ounces (60 g) and can be mailed at minimum cost. The fingerprinting system of the invention is low-cost, fast, convenient and yields excellent prints in a clean, simple, self-administered method.

It is to be realized that only preferred embodiments of the invention have been described and that numerous substitutions, modifications and alterations are permissible without departing from the scope of the invention as defined in the following claims.

## Claims

1. A fingerprint identification system comprising:—

a fingerprint marking composition applicator in the form of a thin packet (41) comprising a vapor-proof enclosure (48) and a porous pad (46) contained within the enclosure (48) impregnated with a controlled amount of marking composition; and

a substrate in the form of a fingerprint card (40) having a portion impregnated with a color developer for the marking composition;

characterised in that the packet (41) is formed on a portion of the card (40).

2. A system as claimed in Claim 1, characterised in that the marking composition contains a color-forming water soluble, metal salt marking compound dissolved in a water-soluble organic solvent.

3. A system as claimed in Claim 2, characterised in that the composition contains 2 to 30 parts by weight of said metal salt, 20 to 100 parts of an organic solvent and 0 to 20 parts of water.

4. A system as claimed in any preceding claim, characterised in that the developer is selected from a polyhydroxy aromatic compound, a quinolinol or mixtures thereof.

5. A system as claimed in Claim 4, characterised in that the salt is an ion chloride and in that the developer comprises 8-hydroxyquinoline and propyl gallate.

6. A system as claimed in Claim 5, characterised in that the ion chloride is a mixture of ferric and ferrous chloride.

7. A system as claimed in any preceding claim, characterised in that the packet (41) is edge-sealed.

8. A system as claimed in any preceding claim, characterised in that the pad (46) is a folded sheet of paper.

9. A system as claimed in Claim 8, characterised in that the pad (46) contains from 100 to 170 pounds (45 to 76.5 kg) of marking solution per 100 pounds (45 kg) of dry paper.

10. A system as claimed in Claim 8 or 9, characterised in that the paper is a creped paper.

11. A system as claimed in any preceding claim, characterised in that the packet (41) is sealed inward from the edge to provide flaps for gripping the packet (41) during opening of the side seams.

## Patentansprüche

1. Fingerabdruckerkennungssystem mit:—

Anbringungsmitteln zur Anbringung einer Färbmasse für einen Fingerabdruck in Form einer dünnen Verpackung (41), die ein dampfdichtes Behältnis (48) und ein poröses Kissen (46) enthält, das innerhalb des Behältnisses enthalten und mit einer vorbestimmten menge an Färbmasse durchtränkt ist; sowie

einem Substrat in Form einer Fingerabdruckkarte (40), wovon ein Abschnitt mit einem Farbentwickler für die Färbmasse durchtränkt ist;

dadurch gekennzeichnet, daß die Verpackung (41) an einem Abschnitt der Karte (40) gebildet ist.

2. System nach Anspruch 1, dadurch gekennzeichnet, daß die Färbmasse eine farbbildende wasserlösliche Metallsalzverbindung enthält, die in einem wasserlöslichen organischen Lösungsmittel gelöst ist.

3. System nach Anspruch 2, dadurch gekennzeichnet, daß die Färbmasse 2 bis 30 Gew.-Teile der genannten Metallsalzverbindung, 20 bis 100 Gew.-Teile eines organischen Lösungsmittels sowie 0 bis 20 Gew.-Teile Wasser enthält.

4. System nach einem der vorhergehenden Ansprüche, dadurch gekennzeichnet, daß der Entwickler eine aromatische Polyhydroxylverbindung, ein Chinolinol oder Gemische davon enthält.

5. System nach Anspruch 4, dadurch gekennzeichnet, daß der Entwickler β-Hydroxyquinolin und Gallussäurepropylester enthält.

6. System nach Anspruch 5, dadurch gekennzeichnet, daß das Einsenchlorid eine Mischung aus Eisen(III)- und Eisen(II)-Chlorid ist.

7. System nach einem der vorhergehenden Ansprüche, dadurch gekennzeichnet, daß die Verpackung (41) am Rand verschweißt ist.

8. System nach einem der vorhergehenden Ansprüche, dadurch gekennzeichnet, daß das Kissen (46) ein zusamengefaltetes Paperblatt ist.

9. System nach Anspruch 8, dadurch gekennzeichnet, daß das Kissen (46) 100 bis 170 Pfund (45 bis 76.5 Kg) der Färblösung je 100 Pfund (45 Kg) trockenes Paper enthält.

10. System nach Anspruch 8 oder Anspruch 9, dadurch gekennzeichnet, daß das Papier ein Kreppapier ist.

11. System nach einem der vorhergehenden Ansprüche, dadurch gekennzeichnet, daß die Verpackung (41) nach innen vom Rand her verschweißt ist, um Klappen zum Efassen der Verpackung (41) beim Öffnen der Randnähte zu bilden.

## Revendications

1. Un système d'identification pour empreinte digitale comprenant:

un applicateur d'une composition de marquage pour empreinte digitale ayant la forme d'un mince paquet (41) comportant une enveloppe étanche à l'eau (48) et un tampon poreux (46) contenu dans l'enveloppe (48) et imprégné d'une quantité spécifiée de composition de marquage et

un substrat ayant la forme d'une carte (40) pour empreinte digitale dont une partie est imprégnée d'une substance de développement de couleur pour réaliser la composition de marquage;

caractérisé en ce que le paquet (41) est formé sur une partie de la carte (40).

2. Un système selon la revendication 1, caractérisé en ce que la composition de marquage contient un composé de marquage hydrosoluble, formant une couleur et à base de sel métallique, et qui est dissous dans un solvant organique hydrosoluble.

3. Un système selon la revendication 2, caractérisé en ce que la composition contient 2 à 30 parties en poids dudit sel métallique, 20 à 100 parties d'un solvant organique et 0 à 20 parties d'eau.

4. Un système selon l'une des précédentes revendications, caractérisé en ce que la substance de développement de la couleur est choisi parmi un composé polyhydroxy-aromatique, un quinolinol ou un mélange de ceux-ci.

5. Un système selon la revendication 4, caractérisé en ce que le sel est un ion chlorure, et en ce que la substance de développement contient du 8-hydroxyquinoline et du gallate de propyle.

6. Un système selon la revendication 5, caractérisé en ce que l'ion chlorure est un mélange de chlorures ferreux et ferrique.

7. Un système selon l'une quelconque des revendications précédentes, caractérisé en ce que le paquet (41) est scellé sur les bords.

8. Un système selon les revendications précédentes, caractérisé en ce que le tampon (46) est une feuille de papier pliée.

9. Un système selon l'une quelconque des revendications précédentes, caractérisé en ce que le tampon (46) contient entre 100 et 170 livres (45 à 76.5 kg) de solution de marquage par 100 livres (45 kg) de papier sec.

10. Un système selon la revendication 8 ou 9, caractérisé en en ce que le papier est du papier crêpe.

11. Système selon l'une quelconque des revendications précédentes, caractérisé en ce que le paquet (41) est scellé à une certaine distance du bord pour constituer des pattes permettant de saisir le paquet (41) au cours de l'ouverture des soudures latérales.

Fig. 1.

Fig. 2.

Fig. 3.

Fig. 5.

Fig. 4.

NAME                                    S.S. NO.

ADDRESS

ORGANIZATION                            SEX

R.T.    R.I.    R.M.    R.R.    R.L.

L.T.    L.I.    L.M.    L.R.    L.L.

1

Fig. 6.

Fig. 7A.

Fig. 7B.

Fig. 7C.

2